# EUROPÄISCHE PATENTSCHRIFT

(11) **EP 0 435 989 B1**
(45) Veröffentlichungstag und Bekanntmachung des Hinweises auf die Patenterteilung: **14.09.1994**
(21) Anmeldenummer: 90910710.4
(22) Anmeldetag: 07.07.1990
(51) Int. Cl.: C07C 255/49, C07C 255/54, C07D 239/26, C07D 239/28, C07D 213/57, C07D 213/84, C07C 253/22, C07C 253/20

(54) **VERFAHREN ZUR HERSTELLUNG VON AROMATISCHEN NITRILEN**
PROCESS FOR THE PREPARATION OF AROMATIC NITRILES
PROCEDE POUR LA FABRICATION DE NITRILES AROMATIQUES

(30) Priorität: 20.07.1989 DE 3923979
(43) Veröffentlichungstag der Anmeldung: 10.07.1991
(73) Patentinhaber: MERCK PATENT GmbH, D-64271 Darmstadt (DE)
(72) Erfinder: PAULUTH, Detlef, D-6105 Ober-Ramstadt (DE); STAHL, Klaus-Peter, W-6100 Darmstadt (DE)
(86) Internationale Anmeldenummer: EP9001104
(87) Internationale Veröffentlichungsnummer: WO9101298

(56) Entgegenhaltungen:
- DE-B- 1 279 020
- US-A- 2 901 504
- US-A- 3 948 968
- PATENT ABSTRACTS OF JAPAN, vol. 12, No. 450, (C-547)(3297), 25 November 1988, & JP-A-63174963

## Beschreibung

Die Erfindung betrifft ein Verfahren zur Herstellung substituierter aromatischer Nitrile durch Umsetzung aromatischer Carbonsäuren mit gasförmigem Ammoniak in Gegenwart eines Dehydratisierungs-Katalysators.

Einfache aromatische Nitrile wie Nicotinsäurenitril und Terephthalonitril sind bedeutende Zwischenprodukte in der organischen industriellen Chemie. Entsprechend substituierte aromatische Nitrile wie z.B. 4-(trans-4-Propylcyclohexyl)-benzonitril (Flüssigkristall) können auch hochveredelte Endprodukte sein.

Eine Übersicht über geeignete Methoden zur Herstellung aromatischer Nitrile ist z.B. in Methoden der Organischen Chemie, Houben-Weyl, Band VIII, zu finden.

Bei den großtechnisch hergestellten aromatischen Nitrilen dominiert als Herstellungsverfahren die Ammonoxidation, bei der Methylaromaten mit Ammoniak und Sauerstoff in Gegenwart eines Katalysators umgesetzt werden. Diese Verfahren sind Gegenstand zahlreicher Patentanmeldungen, wie z.B. DE-OS-18 03 184, DE-OS-19 30 880, FR-OS-15 95 151. So werden z.B. p- und m-Xylol durch Ammonoxidation in die entsprechenden Dinitrile überführt und Nicotinsäurenitril aus 3-Picolin nach diesem Verfahren hergestellt (K. Weissermel und H.-J. Arpe, Industrielle Organische Chemie, 3. Auflage, Seite 422, VCH, 1988).

Daneben sind auch Verfahren zur Herstellung von Phthalodinitril aus bis-Trichlorbenzonitril und Ammoniak in Gegenwart eines Katalysators (EP 250 872 A) und zur Herstellung von Isophthalonitril durch Umsetzung von Isophthaloylchlorid mit Ammoniak in Gegenwart eines Katalysators (EP 241 755 A) bekannt.

Zur Herstellung von aliphatischen Nitrilen, z.B. von Fettsäurenitrilen hat sich die Umsetzung der entsprechenden Säuren mit Ammoniak in Gegenwart eines Katalysators bei Temperaturen zwischen 250 und 550 °C durchgesetzt, vgl. z.B. US 3,661,970; US 3,850,974; US 3,674,708; DD 205 158 und RO 70048.

In den japanischen Patentanmeldungen J5 0013-325 und J5 0013-326 wird neben der Herstellung von aliphatischen Nitrilen durch Umsetzung der entsprechenden Säuren mit Ammoniak in Gegenwart von Cobalt- und Cadmiumsalzen auch die Herstellung von Benzonitril erwähnt.

In der DE-OS 20 20 866 sowie in der darin zitierten japanischen Patentanmeldung J 4 03 29-944 werden Verfahren zur Herstellung von Hydroxy- und Aminobenzonitrilen aus den entsprechenden Benzoesäureestern mittels Ammoniak an Katalysatoren beschrieben.

Dagegen sind die Ausgangsprodukte des erfindungsgemäßen Verfahrens freie, substituierte aromatische Carbonsäuren, deren Substituenten aufgrund der Reaktionsbedingungen zu Isomerisierungs-, Disproportionierungs- oder Transalkyierungsreaktionen neigen.

Die Herstellung von substituierten aromatischen Nitrilen mit aliphatischen Seitenketten, die ggf. auch Heteroatome wie Sauerstoff oder ein Chiralitätszentrum in der Seitenkette und weitere Substituenten am Aromaten (z.B. Halogenatome) enthalten, aus den entsprechenden Säuren unter den obengenannten Bedingungen ist bisher nicht bekannt. Dies ist nicht verwunderlich, da bekannt ist, daß bei Alkylaromaten wie z.B. Xylol im Bereich von 200-260 °C in Gegenwart von Zeolith-Katalysatoren und bei 400-500 °C in Gegenwart von Al₂O₃-SiO₂ sowohl Isomerisierungen als auch Disproportionierungen und Transalkylierungen möglich sind (Weissermel., S. 353). Darüber hinaus sind auch Verkohlungsreaktionen möglich.

Während aliphatische Fettsäuren weder als freie Säure noch als Salz decarboxyliert werden können (z.B. J. March, Advanced Organic Chemistry, S. 562, 1985), ist es bekannt, daß aromatische Carbonsäuren bei erhöhten Temperaturen, z.B. beim Erhitzen mit Kupfer und Kupfersalzen in Chinolin, beim Erhitzen mit starken Säuren und beim Erhitzen ihrer Salze decarboxylieren (z.B. J. March, S. 507).

Außerdem isomerisieren aromatische Carbonsäuren beim Erhitzen in Gegenwart von Zink- und Cadmiumsalzen (z.B. Weissermel, S. 420).

Solche substituierten Nitrile werden im allgemeinen so hergestellt, daß zunächst der Grundkörper des Moleküls aufgebaut wird, der sich aus einem sterisch einheitlichen aliphatischen und dem aromatischen oder heteroaromatischen Teil zusammensetzt. Dann wird durch Bromierung des Aromaten und anschließende Substitution von Br gegen CN (z.B. mit Kupfercyanid) das Nitril hergestellt. Wenn der aromatische Teil bereits eine Carboxylgruppe enthält, wird die Cyano-Gruppe durch eine dreistufige Reaktionssequenz (Säurechlorid, Amid, Nitril) eingeführt. So erhält man z.B. p-(trans,trans-4-Alkylbicyclohexyl)-3-fluorbenzonitrile auf diese Weise in nur 14%iger Ausbeute (z.B. E. Poetsch, Kontakte, 1988, 2, S. 21).

Weitere wichtige Methoden zur Herstellung substituierter aromatischer Nitrile sind die Dehydratisierung der entsprechenden Aldoxime und die Einführung der Nitrilgruppe nach Sandmeyer.

Alle genannten Methoden sind aus industrieller Sicht mit unterschiedlichen Nachteilen behaftet. So entsteht bei der Einführung der Nitrilgruppe mit dem hochgiftigen Kupfercyanid ein aus Kupferbromid und überschüssigem Kupfercyanid zusammengesetzter schlecht zu entsorgender Abfall.

Mehrstufige Verfahren wie die Überführung von Säuren über Säurechlorid und Amid ins Nitril sind kostenintensiv. In Abhängigkeit von den eingesetzten Reagenzien entstehen auch hier problematische Abfälle wie SO₂ und HCl bei Einsatz von Thionylchlorid oder Phosphorsäure und HCl bei Einsatz von POCl₃ oder PCl₅. Entsprechendes gilt für die Dehydratisierung von Aldoximen.

Die Sandmeyerreaktion beinhaltet mehrere gravierende Nachteile. So ist hier ein großer Cyanidüberschuß notwendig. Aufgrund des wasserhaltigen Reaktionsgemisches muß bei Aminen mit einem großen unpolaren Rest in Suspension gearbeitet werden, was sich häufig negativ auf Umsatz und Reaktionsgeschwindigkeit auswirkt. Auch hier fallen erhebliche Mengen an cyanidhaltigen Kupfersalzen als Abfall an.

In der US-A 2 901 404 wird die Herstellung von Phthalsäuredinitril aus Phthalsäure mit Ammoniak beschrieben, wobei die Reaktanden in einer Gasphasenreaktion umgesetzt werden. Bei dieser Reaktion wird die umzusetzende Carbonsäure zusammen mit Ammoniak in der Gasphase über einen Fließbettkatalysator geleitet. Die nach der US-A 2 901 404 erforderlichen Reaktionsbedingungen bedingen zudem einen erheblichen apparativen Aufwand, insbesondere eine Apparatur mit verschiedenen Temperaturbereichen.

Aufgabe der vorliegenden Erfindung war es, ein Herstellungsverfahren für substituierte, aromatische Nitrile zu finden, das die beschriebenen Nachteile der bisherigen Verfahren nicht oder nur in geringem Maß aufweist.

Überraschenderweise wurde nun gefunden, daß sich substituierte Benzoe-, Pyridincarbon-, und Pyrimidincarbonsäuren in hoher Ausbeute durch Umsetzung mit Ammoniak in Gegenwart eines Katalysators bei 200 bis 350 °C in die entsprechenden substituierten aromatischen Nitrile überführen lassen, ohne daS in nennenswertem Ausmaß ausbeutemindernde Nebenreaktionen auftreten.

Bei dieser Reaktion werden aus der Carbonsäure und Ammoniak in situ die entsprechenden Amide hergestellt, welche zum Nitril dehydratisiert werden. Diese Amide können auch direkt im erfindungsgemäßen Verfahren dehydratisiert werden.

Gegenstand der Erfindung ist somit ein Verfahren zur Herstellung substituierter, aromatischer Nitrile durch Umsetzung aromatischer Carbonsäuren oder deren Amide mit gasförmigen Ammoniak in Gegenwart eines Dehydratisierungskatalysators, wobei man einen Ammoniakstrom durch die Schmelze der aromatischen Carbonsäuren oder deren Amide, in der der Katalysator gelöst oder suspendiert ist, leitet.

Der Begriff substituierte aromatische Nitrile umfaßt substituierte Benzonitrile, substituierte Pyridin- und Pyrimidincarbonitrile sowie substituierte und unsubstituierte Cyannaphthaline.

Als Substituenten kommen geradkettige und verzweigte Alkyl- und Oxaalkylgruppen (auch chirale), Cycloalkylgruppen wie Cyclohexyl und Alkyl- und Oxaalkylcyclohexyl, Phenyl-, Alkylphenyl- und Oxaalkylphenylgruppen sowie die entsprechenden Pyridyl- und Pyrimidylgruppen in Frage. Der Phenylring kann neben der Alkylgruppe noch weitere Substituenten wie z.B. Halogene, insbesondere Fluor und Chlor, enthalten.

Die nach dem erfindungsgemäßen Verfahren hergestellten substituierten aromatischen Nitrile sind teilweise bekannt und teilweise neu. Die neuen nach dem erfindungsgemäßen Verfahren hergestellten Verbindungen sind ebenfalls Gegenstand der Erfindung.

Insbesondere können nach dem erfindungsgemäßen Verfahren aromatische Nitrile der Formel I,

R-(A¹-Z¹)ₘ-(A²-Z²)ₙ-Ar-CN I

worin
- R: H, Halogen, -CN, einen unsubstituierten, einen einfach durch CN oder CF₃, oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
- Ar: einen 1,4-Phenylen-, Naphthalin-2,6-diyl-, Pyridin-2,5-diyl- oder Pyrimidin-2,5-diylrest,
wobei die Reste (a), (b) und Ar durch CN oder Halogen substituiert sein können,
- Z¹ und Z²: jeweils unabhängig voneinander -CO-O-, -O-CO-, -CH₂O-, -OCH₂-, -CH₂S-, -SCH₂-, -CH₂CH₂-, -CH=CH-, -C≡C- oder eine Einfachbindung,
- m und n: jeweils unabhängig voneinander 0, 1, oder 2,
bedeuten,
hergestellt werden.

Der Einfachheit halber bedeuten im folgenden Phe eine 1,4-Phenylengruppe, worin auch eine oder zwei CH-Gruppen durch N ersetzt sein können, wobei eine 1,4-Phenylengruppe auch durch ein oder zwei Halogen-Atome und/oder CN-Gruppen substituiert sein können, Cy eine 1,4-Cyclohexylengruppe, worin auch eine oder zwei nicht benachbarte CH₂-Gruppen durch s- und/oder O-Atome ersetzt sein können.

Diese substituierten 1,4-Phenylengruppen sind vorzugsseise 2-Fluor-, 3-Fluor- oder 2,3-Difluorphenylengruppen.

Vor- und nachstehend haben R, A¹, A², Ar, Z¹, Z², m und n die angegebene Bedeutung, sofern nicht ausdrücklich etwas anderes vermerkt ist.

Die nach dem erfindungsgemäßen Verfahren hergestellten Verbindungen der Formel I umfassen demanch diejenigen der Formeln Ia bis Ik:

R-A¹-Phe-CN Ia

R-A¹-Z¹-Phe-CN Ib

R-A¹-A²-Phe-CN Ic

R-A¹-A²-Z²-Phe-CN Id

R-A¹-Z¹-A²-Phe-CN Ie

R-A¹-Z¹-A²-Z²-Phe-CN If

R-A¹-A¹-A²-Phe-CN Ig

R-A¹-Z¹-A¹-A²-Phe-CN Ih

R-A¹-A¹-Z¹-A²-Phe-CN Ii

R-A¹-A¹-A²-Z²-Phe-CN Ij

R-A¹-Z¹-A¹-A²-Z²-Phe-CN Ik.

Darunter sind diejenigen der Formeln Ia, Ib, Ic, Id und Ie besonders bevorzugt.

Von den Verbindungen der Formeln Ia sind diejenigen der Teilformeln Iaa bis Iaf besonders bevorzugt:

Alkyl-Cy-Phe-CN Iaa

Alkyl-Phe-Phe-CN Iab

Alkoxy-Phe-Phe-CN Iac

Alkoxy-Cy-Phe-CN Iad

Alkyl-Cy-CH₂CH₂-Phe-CN Iae

Alkoxy-Cy-CH₂CH₂-Phe-CN Iaf.

In den bevorzugten Verbindungen der vor- und nachstehenden Formeln ist R eine Alkylgruppe mit vorzugsweise 1 bis 10 C-Atomen, eine Alkoxy- oder eine Alkanoyloxygruppe mit vorzugsweise jeweils 1 bis 10 C-Atomen.

Besonders bevorzugte Alkylgruppen sind Hexyl, Pentyl, Butyl, i-Butyl, Propyl, i-Propyl, Methyl und Ethyl, insbesondere Methyl; besonders bevorzugte Alkoxygruppen sind Hexoxy, Pentoxy, i-Butoxy, Propoxy, i-Propoxy, Methoxy und Ethoxy, inbesondere Methoxy; besonders bevorzugte Alkanoyloxygruppen sind Hexanoyloxy, Pentanoyloxy, Butyryloxy, Propionyloxy, Acetyloxy und Formyloxy, insbesondere Acetyloxy.

In den bevorzugten Verbindungen der vor- und nachstehenden Formeln können die Alkylreste, in denen auch eine CH₂-Gruppe (Alkoxy bzw. Oxaalkyl) durch ein O-Atom ersetzt sein kann, geradkettig oder verzweigt sein. Vorzugsweise haben sie 2, 3, 4, 5, 6, 7, 8, 9 oder 10 C-Atome und bedeuten demnach bevorzugt Ethyl, Propyl, Butyl, Pentyl, Hexyl, Heptyl, Octyl, Nonyl, Decyl, Propoxy, Ethoxy, Butoxy, Pentoxy, Hexoxy, Heptoxy, Octoxy, Nonoxy oder Decoxy, ferner auch Undecyl, Dodecyl, Undecoxy, Dodecoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl.

A¹ und A² sind bevorzugt Cy oder Phe. In den Verbindungen der vor- und nachstehenden Formeln bedeutet Phe vorzugsweise eine 1,4-Phenylen- (Ph), eine ein- oder zweifach durch F oder CN substiuierte 1,4-Phenylengruppe (PheX) eine Pyrimidin-2,5-diyl- (Pyr), eine Pyridin-2,5-diyl-(Pyn), eine Pyrazin-3,6-diyl- oder eine Pyridazin-2,5-diyl-Gruppe, insbesondere bevorzugt Ph, PheX, Pyr oder Pyn. Vorzugsweise enthalten die nach dem erfindungsgemäßen Verfahren hergestellten Verbindungen nicht mehr als eine 1,4-Phenylengruppe, worin eine oder zwei CH-Gruppen durch N ersetzt sind. Cy bedeutet vorzugsweise eine 1,4-Cyclohexylengruppe. Insbesondere bevorzugt sind jedoch Verbindungen der Formel I, worin eine der Gruppen A¹ und A² eine in 1- oder 4-Position durch CN substituierte 1,4-Cyclohexylengruppe bedeutet und die Nitrilgruppe sich in axialer Position befindet, d.h. die Gruppe A¹ bzw. A² die folgende Konfiguration aufweist: 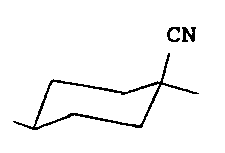
Besonders bevorzugt sind Verbindungen der Formel I und der vorstehenden Teilformeln, die eine Gruppierung -Phe-Phe- enthalten. -Phe-Phe- ist vorzugsweise -Ph-Ph-, -Pyr-Phe- oder -Ph-Pyn-. Besonders bevorzugt sind die Gruppen 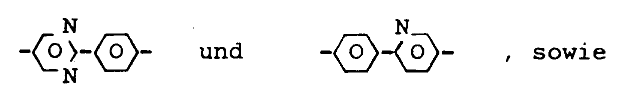
ferner unsubstituiertes oder ein- oder mehrfach durch Fluor substituiertes 4,4'-Biphenylyl.

Insbesondere bevorzugt sind Verbindungen der Formel I und der nachstehenden Teilformeln, die eine 2,3-Difluor-1,4-phenylengruppe enthalten.

Die Gruppen Z¹ und Z² bedeuten jeweils unabhängig voneinander bevorzugt eine Einfachbindung, in zweiter Linie bevorzugt -O-CO-, -CO-O-, -C≡C- oder -CH₂CH₂- Gruppen. Insbesondere bevorzugt sind Verbindungen der Formeln I worin eine Gruppe Z¹-CH₂CH₂- und die andere -O-CO- oder -CO-O- bedeutet.

Verbindungen der vor- und nachstehenden Formeln mit verzweigten Flügelgruppen R¹ können von Bedeutung sein. Verzweigte Gruppen dieser Art enthalten in der Regel nicht mehr als zwei Kettenverzweigungen. R¹ ist vorzugsweise eine geradkettige Gruppe oder eine verzweigte Gruppe mit nicht mehr als einer Kettenverzweigung.

Bevorzugte verzweigte Reste sind Isopropyl, 2-Butyl (= 1-Methylpropyl), Isobutyl (= 2-Methylpropyl), tert.-Butyl, 2-Methylbutyl, Isopentyl (= 3-Methylbutyl), 2-Methylpentyl, 3-Methylpentyl, 4-Methylpentyl, 2-Ethylhexyl, 5-Methylhexyl, 2-Propylpentyl, 6-Methylheptyl, 7-Methyloctyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Der Rest R kann auch ein optisch aktiver organischer Rest mit einem asymmetrischen Kohlenstoffatom sein. Vorzugsweise ist dann das asymmetrische Kohlenstoffatom mit zwei unterschiedlich substituierten C-Atomen, einem H-Atom und einem Substituenten ausgewählt aus der Gruppe Fluor, Alkyl oder Alkoxy mit jeweils 1 bis 5 C-Atomen und CN verknüpft. Der optisch aktive organische Rest R hat vorzugsweise die Formel, 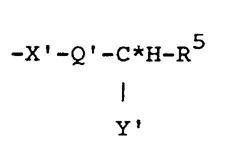
worin
- X': -CO-O-, -O-CO-, -O-CO-O-, -CO-, -O-, -S-, -CH=CH-, -CH=CH-COO- oder eine Einfachbindung,
- Q': Alkylen mit 1 bis 5 C-Atomen, worin auch eine nicht mit X' verknüpfte CH₂-Gruppe durch -O-, -CO-, -O-CO-, -CO-O- oder -CH=CH- ersetzt sein kann, oder eine Einfachbindung,
- Y': CN, F, Methyl oder Methoxy, und
- R⁵: eine von Y' verschiedene Alkylgruppe mit 1 bis 15 C-Atomen, worin auch eine oder zwei nicht benachbarte CH₂-Gruppen durch -S-, -O-, -CO-, -O-CO-, -CO-O- und/oder -CH=CH- ersetzt sein können,
bedeutet.
- X': ist vorzugsweise -CO-O-, -O-CO-, -CH=CH-COO- (trans) oder eine Einfachbindung. Besonders bevorzugt sind -CO-O-/-O-CO- oder die Einfachbindung.
- Q': ist vorzugsweise -CH₂-, -CH₂CH₂-, -CH₂CH₂CH₂- oder eine Einfachbindung, insbesondere bevorzugt eine Einfachbindung.
- Y': ist vorzugsweise CH₃, -CN oder F, insbesondere bevorzugt CN oder F.
- R⁵: ist vorzugsweise geradkettiges oder verzweigtes Alkyl oder Alkoxy mit 1 bis 10, insbesondere mit 1 bis 7, C-Atomen.

Unter den Verbindungen der Formel I sowie Ia bis Ic sind diejenigen bevorzugt, in denen mindestens einer der darin enthaltenen Reste eine der angegebenen bevorzugten Bedeutungen hat.

Geeignete Dehydratisierungs-Katalysatoren sind z.B. Aluminiumoxid, Aluminiumsilicat, Zinkoxid, Cobaltacetat, Borphosphat und Ammoniumphosphat. Die Katalysatorkonzentration liegt bei 0,1 bis 5,0, vorzugsweise 0,2 bis 5,0 Gew.% bezogen auf die umzusetzende Carbonsäure, wenn die Reaktion im Rundkolben durchgeführt wird.

Bei Verwendung eines Reaktionsrohres ist die erforderliche Katalysatormenge entsprechend größer nämlich 0,5 bis 20, vorzugsweise 1,0 bis 11 Gew.% bezogen auf die umzusetzende Carbonsäure. Der Katalysator wird dann bevorzugt in granulierter Form oder auf einem entsprechenden Träger eingesetzt.

Die als Ausgangsstoffe benögtigten substituierten aromatischen Carbonsäuren sind bekannt oder werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

So können aromatische Carbonsäuren z.B. durch Haloformabbau der entsprechenden Arylmethylketone, die durch Friedel-Crafts Acylierung leicht zugänglich sind, hergestellt werden (Houben-Weyl, Band 8, S. 415 (1952)).

Auch durch die Umsetzung von Aromaten mit Phosgen in Gegenwart von Aluminiumchlorid können über die zunächst gebildeten Carbonsäurechloride nach Verseifung die entsprechenden Carbonsäuren erhalten werden. Eine entsprechende Umsetzung mit Oxalylchlorid führt nach Kohlenmonoxidabspaltung ebenfalls zu Carbonsäuren (z.B. Houben-Weyl, Band 8, S. 378 (1952)).

Weiterhin können aromatische Carbonsäuren durch Umsetzung von Halogenaromaten, die durch Halogenisierung der entsprechenden Aromaten leicht zugänglich sind, mit Kohlenmonoxid in Gegenwart von Wasser und Nickel-, Cobalt-, Eisen-, Rhodium- oder Palladiumkatalysatoren hergestellt werden, wobei eine zusätzliche Bestrahlung mit UV-Licht erforderlich sein kann (z.B. Houben-Weyl, Band E 5/Teil 1, S. 310 (1985)).

Schließlich können Halogenaromaten durch Metallierung, z.B. mit Butyllithium oder Magnesium und anschließender Carboxylierung mit Kohlendioxid zu den entsprechenden Carbonsäuren umgesetzt werden (Houben-Weyl, Band E 5/Teil 1, S. 323 (1985)).

Die Reaktionsdurchführung des erfindungsgemäßen Verfahrens ist einfach. So wird bei 200-400 °C, vorzugsweise bei 200-300 °C, insbesondere bei 220-280 °C ein leichter Ammoniakstrom durch die Schmelze der aromatischen Carbonsäure geleitet, in der der Katalysator gelöst (z.B. Zinkoxid) oder suspendiert (z.B. Aluminiumoxid) ist. Mit überschüssigem Ammoniak wird das bei der Reaktion gebildete Wasser ausgetrieben. Das Nitril kann dann durch geeignete Verfahren (z.B. Kugelrohr- oder Dünnfilmdestillation) nach der Umsetzung direkt aus dem Reaktionsgemisch isoliert werden.

Während der Reaktion wird aus der Carbonsäure das entsprechende Amid gebildet, welches in situ dehydratisiert wird. Es ist nach dem erfindungsgemäßen Verfahren auch möglich das entsprechende Carbonsäureamid direkt anstelle der Carbonsäure einzusetzen.

Vorzugsweise werden die Reaktionsgefäße mit einem aufsteigenden beheizbaren Rohr, welches mit einem granulierten Dehydratisierungskatalysator, vorzugsweise granuliertem Aluminiumoxid, insbesondere Compalox® der Fa. Martinswerk BRD, gefüllt ist, versehen.

Des weiteren sind Katalysatoren aus einem granulierten mineralischen Träger ohne katalytische Eigenschaften (z.B. Ton) geeignet, nachdem sie mit der Lösung eines Dehydratisierungs-Katalysators (z.B. 5%ige Cobaltacetatlösung) getränkt und anschließend getrocknet worden sind.

Der Dehydratisierungs-Katalysator im aufsteigenden Rohr ist erforderlich, um gegebenenfalls sublimierendes oder im Ammoniakstrom mitgerissenes Amid zum Nitril umzusetzen.

Die Reaktion kann bei verschiedenen Drucken durchgeführt werden, vorzugsweise bei 1-10 bar, insbesondere bei 1-3 bar.

Unter den genannten Bedingungen findet weder eine Isomerisierung am Aromaten noch in cycloaliphatischen (z.B. cis/trans-Isomerisierung) oder chiralen aliphatischen Seitenketten (z.B. Racemisierung) statt. Auch eine Entoder Transalkylierung konnte nicht beobachtet werden. Als einziges Nebenprodukt wurde das Decarboxylierungsprodukt 1 mit weniger als 2 % nachgewiesen. Der Umsatz liegt zwischen 95 und 99 %. 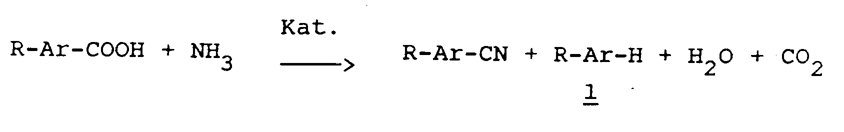
R = Rest, z.B. Alkyl
Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben.

Es bedeuten ferner:
K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Das erfindungsgemäße Verfahren wird vorzugsweise in einem der im folgenden beschriebenen Apparaturen durchgeführt, wobei die Abmessungen dieser Apparaturen unkritisch sind.

### Apparatur 1:

Beheizbarer Rundkolben mit Rührer, Innenthermometer, Gaseinleitungsrohr und aufsteigendem, beheizbarem, 30-50 cm langem Rohr. Das aufsteigende Rohr ist mit einem granulierten Dehydratisierungs-Katalysator gefüllt.

Das beheizte Rohr ist über eine Brücke mit einem Kühler, einer Vorlage (für das bei der Reaktion gebildete Wasser) und einem Blasenzähler verbunden.

### Apparatur 2:

Beheiztes Reaktionsrohr von 30 cm Länge mit 2,5 cm Innendurchmesser, mit Thermoelement, beheiztem Zulaufgefäß, Sumpfblase mit Gaseinleitungsrohr und Abtriebsteil mit absteigendem Kühler. Das Reaktionsrohr ist mit einem granulierten Dehydratisierungskatalysator gefüllt.

Die in den Beispielen aufgeführten Verbindungen sind alle bekannt. Die Identität wurde durch Vergleich der Gas- bzw. HPLC-Chromatogramme, der IR-Spektren und ggf. der Drehwerte mit den entsprechenden Werten von authentischen Mustern sichergestellt.

### Beispiel 1

Eine Mischung aus 192,1 g 4-(trans-4-Pentylcyclohexyl)-benzoesäure (2) und 3,0 g Cobalt-II-acetat wird in Apparatur 1 unter Einleitung von Ammoniak auf 200 °C erhitzt. Man rührt 0,5 h bei dieser Temperatur und erhitzt dann solange weiter auf 270 °C, bis kein Wasser mehr überdestilliert (ca. 6 h). Das Rohprodukt wird bei 170 °C und 0,02 mbar über einen Dünnfilmverdampfer destilliert und anschließend umkristallisiert. Man erhält 169,8 g 4-(trans-4-Pentylcyclohexyl)-benzonitril, K 31 N 55 I.

### Beispiel 2

192,1 g 2 werden mit 1,0 g (0,01 mol) Zinkoxid wie in Beispiel 1 beschrieben umgesetzt und aufgearbeitet. Ausbeute nach 4 h Reaktionszeit 175,4 g 4-(trans-4-Pentylcyclohexyl)-benzonitril, K 31 N 55 I.

### Beispiel 3

192,1 g 2 werden mit 5,0 g Aluminiumoxid wie in Beispiel 1 beschrieben umgesetzt und aufgearbeitet. Ausbeute nach 6 h Reaktionszeit 169,3 g 4-(trans-4-Pentylcyclohexyl)-benzonitril, K 31 N 55 I.

### Beispiel 4

192,1 g 2 werden mit 3,8 g Borphosphat wie in Beispiel 1 beschrieben umgesetzt und aufgearbeitet. Ausbeute nach 6 h Reaktionszeit 153,9 g 4-(trans-4-Pentylcyclohexyl)-benzonitril, K 31 N 55 I.

### Beispiel 5

192,1 g 2 mit 2,0 g Ammoniumdihydrogenphosphat wie in Beispiel 1 beschrieben umgesetzt und aufgearbeitet. Man erhält 174,9 g 4-(trans-4-Pentylcyclohexyl)-benzonitril, K 31 N 55 I.

### Beispiel 6

229,5 g 4'-Dodecyloxy-4-biphenylcarbonsäure werden mit 2,3 g Zinkoxid wie in Beispiel 1 beschrieben umgesetzt. Nach Dünnfilmverdampferdestillation bei 250 °C/0,1 mbar und Umkristallisation erhält man 209,2 g 4-Cyano-4'-dodecyloxybiphenyl, K 70 N 90 I.

### Beispiel 7

194,7 g 4'-Nonyl-4-biphenylcarbonsäure werden wie in Beispiel 5 beschrieben umgesetzt und aufgearbeitet. Man erhält 176,2 g 4-Cyano-4'-nonylbiphenyl, K 42 S 49 N 50 I.

### Beispiel 8

138,7 g Biphenyl-2-carbonsäure werden mit 5,0 g Zinkoxid wie in Beispiel 1 beschrieben umgesetzt. Das Rohprodukt wird bei 140 °C und 0,3 mbar am Dünnfilmverdampfer destilliert. Nach Umkristallisation erhält man 119,6 g 2-Cyanobiphenyl, K 34-35 I.

### Beispiel 9

49,9 g S-4'-(2-Methylbutyl)-biphenyl-4-carbonsäure werden mit 0,5 g Zinkoxid wie in Beispiel 1 beschrieben umgesetzt. Nach Kugelrohrdestillation bei 170 °C und 0,1 mbar erhält man 45,1 g S-4-Cyano-4'-(2-methylbutyl)-biphenyl.

### Beispiel 10

66,8 g 2'-Fluor-4'-(trans-4-propylcyclohexyl)-biphenyl-4-carbonsäure werden mit 0,5 g Ammoniumdihydrogenphosphat wie in Beispiel 1 beschrieben umgesetzt. Nach Filtration und Umkristallisation erhält man 59,7 g 4-Cyano-2'-fluor-4'-(trans-4-propylcyclohexyl)-biphenyl, K 69 N 191 I.

### Beispiel 11

51,3 g 4-(5-Butylpyrimidin-2-yl)-phenyl-1-carbonsäure werden wie in Beispiel 10 beschrieben umgesetzt und aufgearbeitet. Man erhält 44,7 g 4-(5-Butylpyrimidin-2-yl)-benzonitril, K 63 (N 40) I.

### Beispiel 12

34,4 g α-Naphthalincarbonsäure werden wie in Beispiel 1 beschrieben umgesetzt und aufgearbeitet. Man erhält 13,8 g α-Naphthalincarbonitril, K 36-38 I.

### Beispiel 13

30,4 g m-Methoxybenzoesäure werden wie in Beispiel 1 beschrieben umgesetzt und aufgearbeitet. Man erhält 10,4 g m-Methoxybenzonitril, Kp 111-112 °C/13 mm.

Analog werden folgende substituierten Benzonitrile hergestellt:
m-Nitrobenzonitril, mp. 115-117 °C
m-Trifluormethylbenzonitril, Kp. 189-190 °C
4-Methyl-3-brombenzonitril, Kp. 140 °C/0,4 mbar
4-tert.-Butylbenzonitril, mp. 12-14 °C
2,5-Dimethylbenzonitril, mp. 13-15 °C
2,2'-Dicyanobiphenyl, mp. 172-173 °C.
4-Cyanobenzoesäuremethylester, mp. 138-139 °C
4-(4-Hexyloxybenzoyloxy)-benzonitril
4'-Hydroxy-4-cyanobiphenyl

## Patentansprüche

1. Verfahren zur Herstellung substituierter, aromatischer Nitrile durch Umsetzung aromatischer Carbonsäuren oder deren Amide mit gasförmigen Ammoniak in Gegenwart eines Dehydratisierungskatalysators, **dadurch gekennzeichnet, daß** man einen Ammoniakstrom durch die Schmelze der aromatischen Carbonsäuren oder deren Amide, in der der Katalysator gelöst oder suspendiert ist, leitet.

2. Verfahren nach Anspruch 1 zur Herstellung substituierter, aromatischer Nitrile, dadurch gekennzeichnet, daß die Substituenten geradkettige und verzweigte Alkyl- und Oxaalkylgruppen, Cycloalkylgruppen, Alkyl- und Oxaalkylcyclohexyl, Phenyl-, Alkylphenyl- und Oxaalkylphenylgruppen, Pyridyl- und Pyrimidylgruppen sind.

3. Verfahren nach Anspruch 1 zur Herstellung aromatischer Nitrile der Formel I,
R-(A¹-Z¹)ₘ-(A²-Z²)ₙ-Ar-CN I
worin
R Halogen, -CN, einen unsubstituierten, einen einfach durch CN oder CF₃, oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -S-, -O-, -CO-, -CO-O-, -O-CO- oder -O-CO-O- so ersetzt sein können, daß S- und/oder O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
Ar einen 1,4-Phenylen-, Naphthalin-2,6-diyl-, Pyridin-2,5-diyl- oder Pyrimidin-2,5-diylrest,
wobei die Reste (a), (b) und Ar durch CN oder Halogen substituiert sein können,
Z¹ und Z² jeweils unabhängig voneinander -CO-O-, -O-CO-, -CH₂O-, -OCH₂-, -CH₂S-, -SCH₂-, -CH₂CH₂-, -CH=CH-, -C≡C- oder eine Einfachbindung,
m und n jeweils unabhängig voneinander 0, 1, oder 2,
bedeuten.

4. Verfahren nach Anspruch 3 zur Herstellung der Verbindungen der Teilformeln Iaa bis Iaf:
Alkyl-Cy-Phe-CN Iaa
Alkyl-Phe-Phe-CN Iab
Alkoxy-Phe-Phe-CN Iac
Alkoxy-Cy-Phe-CN Iad
Alkyl-Cy-CH₂CH₂-Phe-CN Iae
Alkoxy-Cy-CH₂CH₂-Phe-CN Iaf
worin Alkyl und Alkoxy eine Alkylgruppe oder eine Alkoxygruppe mit jeweils 1 bis 10 C-Atomen bedeutet.

5. Verfahren nach Anspruch 3 zur Herstellung der Verbindungen der Formel I, worin R ein optisch aktiver organischer Rest der Formel 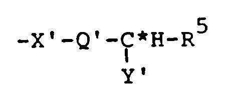 bedeutet,
worin
X' -CO-O-, -O-CO-, -O-CO-O-, -CO-, -O-, -S-, -CH=CH-, -CH=CH-COO- oder eine Einfachbindung,
Q' Alkylen mit 1 bis 5 C-Atomen, worin auch eine nicht mit X' verknüpfte CH₂-Gruppe durch -O-, -CO-, -O-CO-, -CO-O- oder -CH=CH- ersetzt sein kann, oder eine Einfachbindung,
Y' CN, F, Methyl oder Methoxy, und
R⁵ eine von Y' verschiedene Alkylgruppe mit 1 bis 15 C-Atomen, worin auch eine oder zwei nicht benachbarte CH₂-Gruppen durch -S-, -O-, -CO-, -O-CO-, -CO-O- und/oder -CH=CH- ersetzt sein können,
bedeutet.

6. Verfahren nach einem der Ansprüche 1-5, dadurch gekennzeichnet, daß der Dehydratisierungs-Katalysator Aluminiumoxid, Aluminiumsilicat, Zinkoxid, Cobaltacetat, Borphosphat und Ammoniumphosphat ist.

7. Verfahren nach einem der Ansprüche 1 bis 6, dadurch gekennzeichnet, daß das Reaktionsgefäß mit einem aufsteigenden beheizbaren Rohr, welches mit einem granulierten Dehydratisierungskatalysator gefüllt ist, versehen ist.

8. Verfahren nach einem der Ansprüche 1 bis 7, dadurch gekennzeichnet, daß man die Umsetzung bei Temperaturen zwischen 200 und 400 °C durchführt.

9. Verfahren nach einem der Ansprüche 1 bis 8, dadurch gekennzeichnet, daß man 0,1 bis 5,0 Gewichtsprozente an Katalysator, bezogen auf die umzusetzende Carbonsäure, einsetzt.

## Claims

1. Process for the preparation of substituted aromatic nitriles by reacting aromatic carboxylic acids or amides thereof with gaseous ammonia in the presence of a dehydration catalyst, characterized in that a stream of ammonia is passed through the melt of the aromatic carboxylic acids or amides thereof, in which the catalyst is dissolved or suspended.

2. Process according to Claim 1 for the preparation of substituted, aromatic nitriles, characterized in that the substituents are straight-chain and branched alkyl and oxaalkyl groups, cycloalxyl groups, alkyl- and oxaalkylcyclohexyl, phenyl, alkylphenyl and oxaalkylphenyl groups, pyridyl and pyrimidyl groups.

3. Process according to Claim 1 for the preparation of aromatic nitriles of the formula I
R-(A¹-Z¹)ₘ-(A²-Z²)ₙ-Ar-CN I
in which
R is halogen, -CN, an alkyl or alkenyl radical having 1 to 15 C atoms which is unsubstituted, monosubstituted by CN or CF₃ or at least monosubstituted by halogen, it also being possible for one or more CH₂ groups in these radicals to be replaced, in each case independently of one another, by -S-, -O-, -CO-, -CO-O-, -O-CO- or -O-CO-O-, in a manner such that S and/or O atoms are not linked directly to one another,
A¹ and A² are each, independently of one another, a
(a) trans-1,4-cyclohexylene radical in which, in addition, one or more non-adjacent CH₂ groups may be replaced by -O- and/or -S-,
(b) 1,4-phenylene radical in which, in addition, one or two CH groups may be replaced by N,
(c) radicals from the group comprising 1,4-cyclohexenylene, 1,4-bicyclo(2,2,2)octylene, piperidine-1,4-diyl, naphthalene-2,6-diyl, decahydronaphthalene-2,6-diyl and 1,2,3,4-tetrahydronaphthalene-2,6-diyl,
Ar is a 1,4-phenylene, naphthalene-2,6-diyl, pyridine-2,5-diyl or pyrinidine-2,5-diyl radical,
it being possible for the radicals (a), (b) and Ar to be substituted by CN or halogen,
Z¹ and Z² are each, independently of one another, -CO-O-, -O-CO-, -CH₂O-, -OCH₂, -CH₂S-, -SCH₂-, -CH₂CH₂-, -CH=CH-, -C≡C- or a single bond, and
m and n are each, independently of one another, 0, 1 or 2.

4. Process according to Claim 3 for the preparation of the compounds of the sub-formulae Iaa to Iaf:
Alkyl-Cy-Phe-CN Iaa
Alkyl-Phe-Phe-CN Iab
Alkoxy-Phe-Phe-CN Iac
Alkoxy-Cy-Phe-CN Iad
Alkyl-Cy-CH₂CH₂-Phe-CN Iae
Alkoxy-Cy-CH₂CH₂-Phe-CN Iaf.
in which alkyl and alkoxy are alkyl or alkoxy groups, each having 1 to 10 C atoms.

5. Process according to Claim 3 for the preparation of the compounds of the formula I in which R is an optically active organic radical of the formula 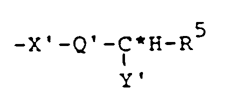 in which
X' is -CO-O-, -O-CO-, -O-CO-O-, -CO-, -O-, -S-, -CH=CH-, -CH=CH-COO- or a single bond,
Q' is alkylene having 1 to 5 C atoms in which, in addition, a CH₂ group which is not linked to X' may be replaced by -O-, -CO-, -O-CO-, -CO-O- or -CH=CH-, or is a single bond,
Y' is CN, F, methyl or methoxy, and
R⁵ is an alkyl group having 1 to 15 C atoms, other than Y', in which, in addition, one or two non-adjacent CH₂ groups may be replaced by -S-, -O-, -CO-, -O-CO-, -CO-O- and/or -CH=CH-.

6. Process according to one of Claims 1-5, characterized in that the dehydration catalyst is aluminium oxide, aluminium silicate, zinc oxide, cobalt acetate, boron phosphate and ammonium phosphate.

7. Process according to one of Claims 1 to 6, characterized in that the reaction vessel is provided with a rising heatable tube which is filled with a granulated dehydration catalyst.

8. Process according to one of Claims 1 to 7, characterized in that the reaction is carried out at temperatures between 200 and 400 °C.

9. Process according to one of Claims 1 to 8, characterized in that 0.1 to 5.0 per cent by weight of catalyst are employed, based on the carboxylic acid to be reacted.

## Revendications

1. Procédé de préparation de nitriles aromatiques substitués par réaction d'acides carboxyliques aromatiques ou de leurs amides avec de l'ammoniac gazeux en présence d'un catalyseur de déshydratation, caractérisé en ce qu'on fait passer un courant d'ammoniac à travers la masse fondue des acides carboxyliques aromatiques ou de leurs amides, dans laquelle le catalyseur est dissous ou en suspension.

2. Procédé selon la revendication 1 de préparation de nitriles aromatiques substitués, caractérisé en ce que les substituants sont des groupes alkyle et oxaalkyle à chaîne droite ou ramifiée, des groupes cycloalkyle, alkyle et oxaalkyl-cyclohexyle, phényle, alkylphényle et oxaalkylphényle, pyridyle et pyrimidyle.

3. Procédé selon la revendication 1 de préparation de nitriles aromatiques de formule I,
R-(A¹ - Z¹)ₘ - (A² - Z²)ₙ - Ar -CN I
dans laquelle
R représente un halogène, -CN, un radical alkyle ou alcényle non substitué, monosubstitué par CN ou CF₃, ou au moins monosubstitué par un halogène, avec de 1 à 15 atomes de carbone, et dans ces radicaux un ou plusieurs groupes CH₂ peuvent également être remplacés indépendamment l'un de l'autre par -S-, -O-, -CO-, -CO-O-, -O-CO- ou -O-CO-O- de manière que les atomes de S et/ou de O ne soient pas reliés directement les uns aux autres,
A¹ et A² représentent chacun indépendamment l'un de l'autre
(a) un radical trans-1,4-cyclohexylène, où également un ou plusieurs groupes CH₂ non voisins peuvent être remplacés par -O- et/ou -S-
(b) un radical 1,4-phénylène, où également un ou deux groupes CH peuvent être remplacés par N,
(c) un radical du groupe 1,4-cyclohexylène, 1,4-bicyclo(2,2,2)-octylène, pipéridine-1,4-diyle, naphtalène-2,6-diyle, décahydro-naphtalène-2,6-diyle et 1,2,3,4-tétra-hydronaphtalène-2,6-diyle,
Aᵣ représente un radical 1,4-phénylène, naphtalène-2,6-diyle, pyridine-2,5-diyle ou pyrimidine-2,5-diyle,
où les radicaux (a), (b) et Ar peuvent être substitués par CN ou un halogène,
Z¹ et Z² représentent chacun indépendamment l'un de l'autre -CO-O-, -O-CO-, -CH₂O-, -OCH₂-, -CH₂S-,
-SCH₂-, -CH₂CH₂-, -CH=CH-, -C≡C- ou une liaison simple,
m et n valent chacun indépendamment l'un de l'autre 0,1 ou 2 ,

4. Procédé selon la revendication 3 de préparation des composés de formules partielles Iaa à Iaf:
Alkyl-Cy-Phe-CN Iaa
Alkyl-Phe-Phe-CN Iab
Alcoxy-Phe-Phe-CN Iac
Alcoxy-Cy-Phe-CN Iad
Alkyl-Cy-CH₂CH₂-Phe-CN Iae
Alcoxy-Cy-CH₂CH₂-Phe-CN Iaf
dans lesquels alkyl et alcoxy représentent un groupe alkyle ou un groupe alcoxy avec à chaque fois de 1 à 10 atomes de carbone.

5. Procédé selon la revendication 3 de préparation des composés de formule I , dans laquelle R représente un radical organique optiquement actif de formule 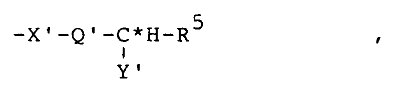 où X' représente -CO-O- , -O-CO- , -O-CO-O- , -CO- , -O- , -S- , -CH=CH- , -CH=CH-COO- ou une liaison simple,
Q' représente un alkylène en C₁ à C₅ , où également un groupe CH₂ non réuni par X' peut être remplacé par -O- , -CO- , -O-CO- , -CO-O- ou -CH=CH- , ou une liaison simple,
Y' représente CN, F, un méthyle ou un méthoxy, et
R⁵ représente un groupe alkyle différent d'Y', en C₁ à C₁₅ , où également un ou deux groupes CH₂ non voisins peuvent être remplacés par -S- , -O- , -CO- , -O-CO- , -CO-O- et/ou -CH=CH- .

6. Procédé selon l'une des revendications 1-5, caractérisé en ce que le catalyseur de déshydratation est l'oxyde d'aluminium, le silicate d'aluminium, l'oxyde de zinc, l'acétate de cobalt, le phosphate de bore ou le phosphate d'aluminium.

7. Procédé selon l'une des revendications 1 à 6, caractérisé en ce que le vase à réaction est muni d'un tuyau montant chauffable rempli d'un catalyseur de déshydratation granulé.

8. Procédé selon l'une des revendications 1 à 7, caractérisé en ce qu'on conduit la réaction à des températures comprises entre 200 et 400°C.

9. Procédé selon l'une des revendications 1 à 8, caractérisé en ce qu'on utilise de 0,1 à 5,0 % en poids de catalyseur, par rapport à l'acide carboxylique à faire réagir.
